(19) Europäisches Patentamt
European Patent Office
Office européen des brevets

(11) EP 0 826 375 B1

(12) EUROPÄISCHE PATENTSCHRIFT

(45) Veröffentlichungstag und Bekanntmachung des Hinweises auf die Patenterteilung:
**08.12.2004 Patentblatt 2004/50**

(51) Int Cl.⁷: **A61K 47/24**, A61K 38/46

(21) Anmeldenummer: **97114330.0**

(22) Anmeldetag: **20.08.1997**

(54) **Verwendung von komplexen Lipiden als stabilisierende Zusätze zu pharmazeutischen Zubereitungen von Verdauungsenzymgemischen**

Use of complex lipids as stabilizing additives for pharmaceutical preparations of digestive enzymes

Utilisation de lipides complexes comme additifs stabilisants de préparations pharmaceutiques de mélanges d'enzymes digestifs

(84) Benannte Vertragsstaaten:
**AT BE CH DE DK ES FI FR GB GR IE IT LI LU NL PT SE**
Benannte Erstreckungsstaaten:
**LT LV**

(30) Priorität: **28.08.1996 DE 19634752**
**12.06.1997 DE 19724845**

(43) Veröffentlichungstag der Anmeldung:
**04.03.1998 Patentblatt 1998/10**

(73) Patentinhaber: **Solvay Pharmaceuticals GmbH**
**30173 Hannover (DE)**

(72) Erfinder: **Galle, Manfred**
**30916 Isernhagen (DE)**

(74) Vertreter: **Gosmann, Martin, Dr. et al**
**Solvay Pharmaceuticals GmbH,**
**Patentabteilung**
**Postfach 220**
**30002 Hannover (DE)**

(56) Entgegenhaltungen:
**US-A- 3 991 180** **US-A- 5 374 657**

- **PATENT ABSTRACTS OF JAPAN & JP 59 169491 A (DUSKIN FRANCHISE), 25. September 1984 (1984-09-25)**
- **CHEMICAL ABSTRACTS, vol. 99, no. 24, 12. Dezember 1983 (1983-12-12) Columbus, Ohio, US; abstract no. 200535, FUJIMOTO PHARMACEUTICAL CO., LTD., JAPAN: "Capsules containing stable digestive enzymes" XP002199200 & JP 58 148814 A (FUJIMOTO PHARMACEUTICAL CO., LTD., JAPAN) 5. September 1983 (1983-09-05)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 826 375 B1

Printed by Jouve, 75001 PARIS (FR)

## Beschreibung

Die vorliegende Erfindung betrifft die Verwendung von komplexen Lipiden als gegen eine Abnahme von lipolytischer Aktivität unter dem Einfluß von Feuchtigkeit stabilisierende Zusätze zu wasserlöslichen pharmazeutischen Zubereitungen von Verdauungsenzymgemischen, welche Protease/Lipase-Gemische, insbesondere Pankreatin, enthalten und welche für die Herstellung von wäßrigen Lösungen zur kontinuierlichen Einführung in den gastrointestinalen Trakt mittels Sonden geeignet sind. Weiterhin betrifft die Erfindung wasserlösliche pharmazeutische Zubereitungen von Lipasen und Proteasen enthaltenden Verdauungsenzymgemischen, insbesondere von pankreatinhaltigen Verdauungsenzymgemischen, welche durch komplexe Lipide gegen eine Abnahme von lipolytischer Aktivität unter dem Einfluß von Feuchtigkeit stabilisiert sind und welche zur Herstellung von in den gastrointestinalen Trakt bei Säugetieren oder Menschen mittels Sonde einführbaren wäßrigen Lösungen geeignet sind.

Bei Säugetieren, insbesondere Menschen, kann ein Mangel an Verdauungsenzymen auftreten, beispielsweise hervorgerufen durch krankhafte Veränderung des Pankreas aufgrund chronischer Pankreatitis, Verdauungsinsuffizienz nach Magenoperationen, Leberoder Gallenerkrankungen. Es ist bereits bekannt, daß man derartige Mangelerscheinungen durch die Gabe von nicht-körpereigenen pankreatinhaltigen Verdauungsenzymgemischen, wie z. B. Pankreasenzymen, insbesondere Pankreatin, welches gegebenenfalls noch Lipasezusätze enthalten kann, behandeln kann. Die Pankreasenzyme werden üblicherweise in Form fester Zubereitungen oral verabreicht. Damit bei der oralen Einnahme die verabreichten Enzymgemische nicht unerwünscht bereits im Magen durch dort vorhandene Magensäure und proteolytische Enzyme, wie Pepsin, irreversibel denaturiert werden, ist es nötig, die Enzymgemische mit einem magensaftresistenten Überzug zu versehen. Ein derartiger Überzug ermöglicht die Passage der intakten Enzymgemische durch den Magen bis zu ihrem Wirkort, dem Duodenum, wo durch die dort herrschenden neutralen bis leicht alkalischen Bedingungen die Schutzschicht abgebaut wird und die Enzyme freigesetzt werden. Wie die körpereigenen Pankreasenzyme des gesunden Menschen können die oral zugeführten Enzyme dort ihre enzymatischen Wirkungen, insbesondere amylolytische, lipolytische sowie proteolytische Aktivitäten entfalten.

Solche mit einem magensaftresistenten Film überziehbaren festen Pankreatinformulierungen in Form von Mikropellets werden z. B. in der DOS 42 27 385.4 beschrieben.

Weiterhin wird in der Literatur die Verwendung von Phosholipiden zum Schutz von Enzymen gegenüber Oxidationsmitteln beschrieben. So offenbart das Dokument JP 59 169 491 die Ummantelung oder Beschichtung jeweils separat zu betrachtender Reinenzyme, beispielsweise einer Protease, Lipase oder Amylase, mit einem Phospholipid. In dem erhaltenen, getrockneten Enzym/Phospholipidgemisch sind die Enzyme gegen Oxidation geschützt. Insbesondere wird als möglicher Oxidationsstress der Kontakt der Enzyme mit einem Peroxid angeführt.

Für Patienten mit Verdauungsinsuffizienz, insbesondere bettlägerige Patienten mit länger anhaltender Verdauungsinsuffizienz wie beispielsweise chronischer Pankreasinsuffizienz, wäre die Verabreichung der nicht-körpereigenen Verdauungsenzyme auch über einen längeren Zeitraum in flüssiger Form, beispielsweise durch kontinuierliche Applikation mittels Sonde, anstelle von festen Darreichungsformen wünschenswert.

Flüssige Darreichungsformen von pankreatinhaltigen Verdauungsenzymgemischen, insbesondere von Pankreatin, konnten bisher nicht zur Verfügung gestellt werden, da flüssige wäßrige Zubereitungen derartiger Enzymgemische nicht über längere Zeit stabil sind. Es zeigt sich, daß insbesondere die Aktivität der im Gemisch enthaltenen Lipasen durch den proteolytischen Angriff der ebenfalls im Gemisch enthaltenen Proteasen wie Trypsin oder Chymotrypsin in Gegenwart von Wasser rasch abnimmt. So kann es je nach äußeren Bedingungen (Temperatur, pH-Wert) in wäßrigen Pankreatin-Zubereitungen innerhalb von kürzester Zeit zu einem weitgehenden Verlust der Lipaseaktivität kommen.

Um zur kontinuierlichen Einführung in den gastrointestinalen Trakt durch Applikation mittels Sonde geeignet zu sein, müssen wäßrige Lösungen von Lipasen und Proteasen enthaltenden Verdaungsenzymgemischen, insbesondere von pankreatinhaltigen Verdauungsenzymgemischen, über einen Zeitraum von mehreren Stunden, beispielsweise 8 Stunden, stabil sein. Insbesondere dürfen in den Lösungen keine die Sonde verstopfenden Partikel entstehen oder enthalten sein. Eine wesentliche Forderung an derartige Lösungen ist, daß eine möglichst hohe und gleichbleibende Aktivität sämtlicher darin enthaltener Verdauungsenzyme über den gesamten Verabreichungszeitraum aufrechterhalten bleibt. Weiterhin ist es für zur kontinuierlichen gastrointestinalen Applikation geeignete Lösungen notwendig, daß diese frei von Keimwachstum, also beispielsweise gegen Keimwachstum konserviert, vorzugsweise steril, bereitgestellt werden können.

Aufgabe der Erfindung war es daher, gegen den Verlust lipolytischer Aktivität unter dem Einfluß von Feuchtigkeit stabilisierte, Lipasen und Proteasen enthaltende wasserlösliche pharmazeutische Zubereitungen von Verdauungsenzymgemischen, welche in wäßrigem Medium gelöst über einen längeren Zeitraum stabil bleiben, zur Verfügung zu stellen.

Gegenstand der Erfindung ist die Verwendung von komplexen Lipiden als gegen eine Abnahme von lipolytischer Aktivität unter Feuchtigkeitseinfluß stabilisierende Zusätze in wasserlöslichen pharmazeutischen Zubereitungen von Lipasen und Proteasen enthaltenden Verdauungsenzymgemischen, insbesondere pankreatinhaltigen Verdauungsenzymgemischen, welche für die Herstellung von wäßrigen Lösungen zur kontinuierlichen Einführung in den gastrointestinalen Trakt mittels Sonde geeignet sind wobei die komplexen lipide ausgewählt sind aus der Gruppe der Phospholipide, Glycolipide und deren Gemische. Gegenstand der Erfindung sind ferner derart stabilisierte wasserlösliche pharmazeutische Zubereitungen von Verdauungsenzymgemischen. Ebenfalls Gegenstand der Erfindung sind Kits zur Herstellung von für die kontinuierliche Sondenapplikation geeigneten wäßrigen Lösungen von Verdauungsenzymgemischen.

Komplexe Lipide, welche sich erfindungsgemäß als stabilisierende Zusätze eignen, sind in der Regel in Aceton unlöslich. Zu ihnen zählen insbesondere die phosphorhaltigen und kohlehydratfreien Phospholipide sowie die kohlehydrathaltigen und nichtphosphorhaltigen Glycolipide und deren Gemische. Zweckmäßig werden nur Phospholipide oder Phospholipide und Glycolipide enthaltende Gemische verwendet.

Als Phospholipide, welche erfindungsgemäß als stabilisierende Zusätze zu Lipasen und Proteasen enthaltenden Verdauungsenzymgemischen, insbesondere pankreatinhaltigen Verdauungsenzymgemischen, verwendet werden können, eignen sich insbesondere Salze von Anionen der allgemeinen Formel I

(s. Formel I)

worin

- $R^1$ Wasserstoff oder einen Alkanoylrest mit 10 - 25 Kohlenstoffatomen, dessen Kohlenwasserstoffrest gegebenenfalls 1 - 4 Doppelbindungen enthalten kann, bedeutet,
- $R^2$ Wasserstoff oder einen Alkanoylrest mit 10 - 25 Kohlenstoffatomen, dessen Kohlenstoffrest gegebenenfalls 1 - 4 Doppelbindungen enthalten kann, bedeutet, oder, wenn $R^1$ nicht für Wasserstoff steht, auch Wasserstoff bedeuten kann,
- $R^3$ Wasserstoff, eine niedere Alkylgruppe, welche durch Amino, niederes Trialkylammonium, eine an ein eine Aminofunktion tragendes Kohlenstoffatom gebundene Carboxylgruppe oder eine durch Hydroxy substituierte Cycloalkylgruppe substituiert sein kann, bedeutet,
- $R^4$ Wasserstoff oder eine Kohlenwasserstoffkette mit 10 - 25 Kohlenstoffatomen, die gegebenenfalls 1 - 4 Doppelbindungen enthalten kann, bedeutet,
- A für Sauerstoff oder NH steht,

mit einem physiologisch verträglichen Kation.

Als physiologisch verträgliche Kationen eignen sich Ammoniumionen, Alkali- oder Erdalkalimetallkationen, vorzugsweise Natrium, Kalium oder Calcium sowie weitere physiologisch verträgliche ein- oder mehrwertige Kationen. Sofern $R^3$ ein Stickstoffatom enthält, kann dieses ein quartäres Ammoniumion bilden, welches ebenfalls als Kation dienen kann, so daß nach außen ungeladene innere Salze gebildet werden.

Sofern in den Verbindungen der Formel I die Reste $R^1$ und/oder $R^2$ für einen Alkanoylrest stehen, kann dieser geradkettig oder verzweigt sein und ist in der Regel unverzweigt und enthält 10 - 25, vorzugsweise 16 - 20 Kohlenstoffatome. Gegebenenfalls kann der Alkanoylrest bis zu 4 Doppelbindungen enthalten. Als Alkanoylreste können insbesondere Reste langkettiger Fettsäuren, wie Nervonsäure, Lignocerinsäure, Palmitinsäure, Palmitoleinsäure, Stearinsäure, Ölsäure, Linolsäure, Linolensäure, Arachinsäure oder Arachidonsäure vorkommen.

Sofern der Substituent $R^3$ eine niedere Alkylgruppe bedeutet oder enthält, kann diese geradkettig oder verzweigt sein und insbesondere 1 bis 4, bevorzugt 1 bis 2 Kohlenstoffatome enthalten. Sofern $R^3$ eine durch Hydroxy substituierte Cycloalkylgruppe bedeutet, kann diese 3 bis 6 Kohlenstoffatome enthalten und ein- oder mehrfach durch Hydroxy substituiert sein. Bevorzugt enthält die Cycloalkylgruppe 5 bis 6 Kohlenstoffatome, die jeweils durch Hydroxy substituiert sein können.

Der Rest $R^3O$ stellt bevorzugt Hydroxy oder einen durch Veresterung eines ein- oder mehrwertigen Alkoholes mit der Phosphatgruppe entstandenen Alkoxyrest dar, wobei der ein-oder mehrwertige Alkohol ausgewählt ist aus der Gruppe bestehend aus Aminoethanol, Cholin, Serin, Glycerin und myo-Inositol.

Sofern der Rest $R^4$ eine Kohlenwasserstoffkette darstellt, kann diese geradkettig oder verzweigt sein und ist in der Regel unverzweigt und enthält 10 - 25, bevorzugt 12 - 20, besonders bevorzugt 15 Kohlenstoffatome. Gegebenenfalls kann die Kohlenwasserstoffkette bis zu 4, bevorzugt 2, besonders bevorzugt 1 Doppelbindung enthalten.

Der Rest A kann für Sauerstoff oder die NH-Gruppe stehen.

Bevorzugt kommen als Phospholipide Phoshpatidsäure (1,2-Diacyl-sn-glycerol-3-phosphorsäure), Phosphatidylcholin (1,2-diacyl-sn-glycerol-3-phosphorylcholin), Phosphatidylethanolamin (1,2-Diacyl-sn-glycerol-3-phosphorylethanolamin), Phosphatidylserin (1,2-diacyl-sn-glycerol-3-phosphorylserin) und Phosphatidylinosit (1,2-Diacyl-sn-glycerol-3-phosphorylinosit) in Frage und für den Fall, daß die Phospholipide aus tierischen Herkunftsquellen stammen, wie beispielsweise Hühnerei, auch Sphingomyelin, sowie Gemische dieser Verbindungen. Die besagten 1,2-Diacylphospholipide können unter gewissen Bedingungen, etwa dem enzymatischen Einfluß einer Phospholipase, partiell hydrolysiert werden. Je nach der Natur der Phospholipase können dabei die Reste $R^1$, $R^2$, $R^3$ oder auch $[R^3OPO_2]^-$ der 1,2-Diacylphospholipide durch Wasserstoff ersetzt werden. Wenn mindestens einer der genannten Molekülreste je Phospholipidmolekül hydrolysiert wird, dann entstehen sogenannte Lysophospholipide, insbesondere Lysophosphatidylcholin, Lysophosphatidylethanolamin, Lysophosphatidylinosit, Lysophosphatidylserin und Lysophosphatidsäure. Auch diese Lysophospholipide eignen sich als stabilisierende Zusätze zu Lipasen und Proteasen enthaltenden Verdauungsenzymgemischen, insbesondere pankreatinhaltigen Verdauungsenzymgemischen, im Sinne der Erfindung.

Als Glycolipide können vor allem in Pflanzen vorkommende, sogenannte Phytoglycolipide der allgemeinen Formel II

(s. Formel II)

worin

| | |
|---|---|
| $R^5$ und $R^6$ | unabhängig voneinander jeweils einen Alkanoylrest mit 10 - 25 Kohlenstoffatomen bezeichnet, dessen Kohlenwasserstoffrest gegebenenfalls 1 - 4 Doppelbindungen enthalten kann, oder Wasserstoff bedeutet, wobei aber nicht $R^5$ und $R^6$ gleichzeitig Wasserstoff bedeuten können, und |
| $R^7$ | einen Mono- oder Disaccharidrest bedeutet, dessen Saccharidbausteine ausgewählt sind aus der Gruppe bestehend aus D-Fructosyl, D-Galactosyl, D-Glucosyl und D-Mannosyl |

sowie deren Gemische verwendet werden.

Sofern in den Verbindungen der Formel II $R^5$ und $R^6$ einen Alkanoylrest darstellen, ist dieser verzweigt oder unverzweigt und ist in der Regel unverzweigt und enthält 10 - 25, vorzugsweise 16 - 20 Kohlenstoffatome. Gegebenenfalls kann der Alkanoylrest bis zu 4 Doppelbindungen enthalten. Als Alkanoylreste kommen insbesondere Reste langkettiger Fettsäuren, wie Palmitinsäure, Palmitoleinsäure, Stearinsäure, Ölsäure, Linolsäure, Linolensäure oder Arachinsäure in Frage.

Die Reste $R^7$ stellen Mono- oder Disaccharidreste dar, die aus den Zuckermolekülen D-Galactose, D-Glucose, D-Mannose oder D-Fructose aufgebaut sein können. Besonders bevorzugt hat $R^7$ die Bedeutung D-Galactose (dann handelt es sich um Monogalactosyl-Diglyderide, MGDG) oder Digalactose (dann handelt es sich um Digalactosyl-Diglyceride, DGDG; 1,2-Diacyl-[$\alpha$-D-galactosyl-(1 $\rightarrow$ 6)-$\beta$-D-galactosyl-(1 $\rightarrow$ 3)]-sn-glycerine).

Die Phospholipide der allgemeinen Formel I und die Glycolipide der allgemeinen Formel II besitzen am mittleren Kohlenstoffatom des Glycerin-Grundkörpers jeweils ein Chiralitätszentrum und können R- oder S-konfiguriert sein. Für den Zweck der Erfindung können die einzelnen stereoisomeren Formen der Verbindungen der Formel I und/oder der Formel II sowie die entsprechenden Gemische verwendet werden.

Für die erfindungsgemäße Stabilisierung der pharmazeutischen Zubereitungen von Lipasen und Proteasen enthaltenden Verdauungsenzymgemischen, insbesondere pankreatinhaltigen Verdauungsenzymgemischen, haben sich solche Lipidgemische als günstig erwiesen, wie sie aus natürlichen Quellen gewonnen werden können und welche Gemische verschiedener Phospholipide und gegebenenfalls verschiedener Glycolipide darstellen. Als bevorzugte Beispiele solcher natürlichen Lipidgemische seien Lecithine genannt. Quellen solcher natürlicher Lecithine können insbesondere Pflanzen, wie Sojabohnen, Sonnenblumen, Raps, Mais oder Erdnüsse sowie Tiere oder Tierprodukte, wie Eigelb oder Hirnsubstanz, aber auch Mikroorganismen sein. Lecithine natürlicher Herkunft sind von verschiedenen Anbietern allgemein kommerziell erhältlich.

Von den pflanzlich gewonnenen Lecithinen ist besonders Sojalecithin, insbesondere an Phospholipiden angereichertes Sojalecithin, wie beispielsweise Sojalecithin mit einem Gehalt an Phospholipiden von ca. 98 %, für den Zweck der Erfindung geeignet. Unbehandelte, nicht angereicherte pflanzliche Lecithine enthalten in der Regel einen gewissen Anteil an Phytoglycolipiden. Natürlich gewonnene Lecithine sind Gemische verschiedener Phospholipide und im Falle pflanzlichen Ursprungs auch Glycolipide, deren Zusammensetzung nicht einheitlich ist, sondern in Abhängigkeit von ihrer Herkunft schwanken kann. So können neben den oben bereits genannten Bestandteilen noch weitere Phospholipide in untergeordneten Mengen enthalten sein. Tabelle 1 dient dazu, durchschnittliche Zusammensetzungen einiger unbehandelter, nicht angereicherter handelsüblicher Lecithine natürlicher Herkunft zu veranschaulichen.

Tabelle 1

| Zusammensetzung einiger Lecithine (%) | | | | |
|---|---|---|---|---|
| | **Soja-Lecithin** | **Raps-Lecithin** | **Erdnuß-Lecithin** | **Ei-Lecithin** |
| Phosphatidylcholin | 22 | 37 | 23 | 73 |
| Phosphatidylethanolamin | 23 | 29 | 8 | 17 |
| Phosphatidylserin | 2 | -- | -- | -- |
| Phosphatidylinosit | 20 | 14 | 17 | 1 |
| Phosphatidsäure | 5 | -- | 2 | -- |
| Sphingomyelin | -- | -- | -- | 3 |
| Phytoglykolipide | 13 | 20 | 38 | 0 |
| Andere Phospholipide | 12 | -- | 12 | -- |

Die erfindungsgemäß stabilisierten pharmazeutischen Zubereitungen enthalten vorzugsweise pankreatinhaltige Verdauungsenzymgemische.

Im Rahmen der vorliegenden Erfindung wird unter Pankreatin aus Säugetierpankreas isoliertes Pankreatin verstanden, dessen Gehalt an aktiven Proteasen gegebenenfalls durch autolytische Spaltung der darin ursprünglich enthaltenen Proteasen-Zymogene erhöht wurde.

Bevorzugt kann es sich bei den pankreatinhaltigen Verdauungsenzymgemischen in den erfindungsgemäß stabilisierten pharmazeutischen Zubereitungen um aus Säugetierpankreas gewonnenes Pankreatin, insbesondere Schweinepankreatin handeln, welches ein Gemisch verschiedener Verdauungsenzyme darstellt. Säugetierpankreatin, das sich als Verdauungshilfsmittel für die humane Ernährung eignet, insbesondere Pankreatin aus Schweinepankreas, enthält für menschliche Bedürfnisse Lipasen nicht immer in ausreichender Menge. Daher kann solchen Pankreatinpräparaten zusätzliche, beispielsweise aus Mikroorganismen gewonnene Lipase zugesetzt werden. Auch die derart erhaltenen Pankreatin/Lipase-Gemische stellen geeignete Enzymgemische dar.

In aus Säugetieren isoliertem Pankreatin liegen die Proteasen, wenn das Pankreatin keiner weiteren Vorbehandlung unterworfen wurde, üblicherweise zum größten Teil in Form einer proteolytisch inaktiven Vorstufe, den Zymogenen, vor. Daher kann es zweckmäßig sein, für pharmazeutische Zwecke das Rohpankreatin, welches auf an sich bekannte Weise durch geeignete Fällungsprozesse aus Pankreas erhalten wurde, noch einer hydrolytischen Behandlung (Autolyse) zu unterwerfen. Bei dieser Behandlung werden Zymogene in aktive Proteasen überführt. In diesem autolytisch vorbehandelten Pankreatin (im folgenden als F-Pankreatin abgekürzt) liegt ein besonders hoher Gehalt an aktiven Proteasen vor, so daß diese F-Pankreatine bezüglich ihrer lipolytischen Aktivität besonders gefährdet sind. Die erfindungsgemäße Verwendung komplexer Lipide eignet sich besonders gut zur Stabilisierung der lipolytischen Aktivität in F-Pankreatin enthaltenden pharmazeutischen Zubereitungen. Dabei ist überraschend, daß die Stabilisierung der Lipaseaktivität nicht durch eine Desaktivierung der im Gemisch enthaltenen aktiven Proteasen erfolgt und die derart stabilisierten Enzymgemische daher sowohl amylolytische und lipolytische, als auch proteolytische Aktivität aufweisen.

Die pankreatinhaltigen Verdauungsenzymgemische in den erfindungsgemäß zu schützenden pharmazeutischen Zubereitungen können neben Pankreatin zusätzlich solche Lipasen enthalten, wie sie in Pflanzen oder Mikroorganismen enthalten sind. Lipasen aus Mikroorganismen können solche sein, die aus Bakterien oder Pilzkulturen wie Schimmelpilzen, beispielsweise des Stammes Rhizopus, gewonnen werden.

Als zusätzliche Proteasenbestandteile können pankreatinhaltigen Verdauungsenzymgemischen in den erfindungsgemäß zu schützenden pharmazeutischen Zubereitungen noch andere tierische und pflanzliche Proteasen sowie insbesondere aus Mikroorganismen wie Bakterien oder Pilzkulturen wie Schimmelpilzen, beispielsweise des Stammes Aspergillus, gewinnbare Proteasen zugesetzt werden.

Pankreatinfreie Verdauungsenzymgemische können als Lipasen solche aus Pflanzen oder Mikroorganismen enthalten. Lipasen aus Mikroorganismen können solche sein, die aus Bakterien oder Pilzkulturen wie Schimmelpilzen, beispielsweise des Stammes Rhizopus, gewonnen werden. Als in pankreatinfreien Verdauungsenzymgemischen enthaltene Proteasen kommen tierische oder pflanzliche Proteasen, sowie insbesondere aus Mikroorganismen wie Bakterien oder Pilzkulturen wie Schimmelpilzen, beispielsweise des Stammes Aspergillus, gewinnbare Proteasen in Frage.

Erfindungsgemäße pharmazeutische Zubereitungen können neben Verdauungsenzymgemischen und komplexen Lipiden welche ausgewählt sind aus der Gruppe der Phospholipide, Glycolipide und deren Gemische zusätzlich wasserlösliche pharmazeutische Hilfsstoffe und/oder Zusatzstoffe enthalten. Beispielsweise können Trägerstoffe wie

Kohlenhydrate, beispielsweise Mannit, oder lösliche Proteine sowie Konservierungsmittel enthalten sein.

Pharmazeutische Zubereitungen von pankreatinhaltigen Verdauungsenzymgemischen im Sinne der Erfindung können wasserlösliche Pulver sein, welche neben Pankreatin, insbesondere F-Pankreatin, komplexe Lipide welche ausgewählt sind aus der Gruppe der Phospholipide, Glycolipide und deren Gemische in einer zur Stabilisierung der lipolytischen Aktivität unter Feuchtigkeitseinfluß ausreichenden Menge und gegebenenfalls zusätzlich an sich bekannte wasserlösliche Hilfs- und/oder Zusatzstoffe enthalten.

Zur Herstellung wasserlöslicher Pulver können die Enzymgemische weitgehend von wasserunlöslichen Bestandteilen befreit werden. Zu diesem Zweck kann eine wäßrige Zubereitung von F-Pankreatin mittels geeigneter, an sich bekannter Verfahren zur Feststoffabtrennung, beispielsweise durch Zentrifugation oder Filtration, von ungelösten Feststoffen befreit werden und die erhaltenen Lösungen, gegebenenfalls nach Zugabe weiterer Hilfs- und/oder Zusatzstoffe, nach an sich bekannten Methoden, beispielsweise durch Sterilfiltration, sterilisiert werden. Anschließend können die Inhaltsstoffe der erhaltenen klaren, gegebenenfalls sterilen Lösungen durch an sich bekannte Trocknungsverfahren, beispielsweise durch Gefriertrocknung, wieder als Feststoffe erhalten werden. Die derart erhaltenen Zubereitungen eignen sich zur Herstellung von über mehrere Stunden, z. B. bis zu acht Stunden, stabilen, gegebenenfalls keimwachstumsfreien Lösungen, welche sich zur kontinuierlichen, gleichmäßigen gastrointestinalen Applikation an Patienten, z. B. per Sonde eignen.

Unter einer kontinuierlichen Applikation wird eine im wesentlichen ununterbrochene Verabreichung von die erfindungsgemäßen pharmazeutischen Zubereitungen enthaltenden, gegebenenfalls sterilen wäßrigen Lösungen über einen Zeitraum von etwa einer Stunde bis zu mehreren Stunden, beispielsweise 8 Stunden, etwa über Nacht, verstanden. Die kontinuierliche Applikation der Lösungen kann vorteilhaft über in den Verdauungstrakt, beispielsweise in den Magen oder den Dünndarm, eingebrachte Sonden erfolgen.

Die Wirksamkeit des Lecithinzusatzes ist weitgehend unabhängig vom relativen Lipase/Proteaseverhältnis im Enzymgemisch. Geeignet sind beispielsweise Enzymgemische, worin das Verhältnis Lipase/Gesamtprotease - gemessen am Verhältnis der jeweiligen Aktivitäten, welche nach den Bestimmungen der "Federation Internationale Pharmaceutique" (im folgenden mit FIP abgekürzt) ermittelt wurden (siehe R. Ruyssen und A. Lauwers, Pharmaceutical Enzymes, Scientific Publishing Company, Gent 1978, S. 74 - 82, im folgenden als "Lauwers" zitiert) und im folgenden in relativen Aktivitätseinheiten, abgekürzt mit FIP-E/g, angegeben werden, von etwa 5:1 bis etwa 30:1 betragen kann.

Grundsätzlich bewirkt der erfindungsgemäße Zusatz komplexer Lipide welche aus der Gruppe der Phospholipide, Glycolipide und deren Gemische ausgewählt sind, zu pharmazeutischen Zubereitungen von Lipasen und Proteasen enthaltenden Verdauungsenzymgemischen, insbesondere pankreatinhaltigen Verdauungsenzymgemischen, eine Stabilisierung gegen eine Abnahme der lipolytischen Aktivität unter dem Einfluß von Feuchtigkeit. Unter Feuchtigkeit soll im wesentlichen wäßrige Feuchtigkeit verstanden werden, die von einem sehr geringen Feuchtigkeitsgehalt des Verdauungsenzymgemisch-Pulvers bis hin zu einer wäßrigen Zubereitung dieses Pulvers reichen kann.

Ein erfindungsgemäßer Zusatz komplexer Lipide welche ausgewählt sind aus der Gruppe der Phospholipide, Glycolipide und deren Gemische, erweist sich bereits bei der Gewinnung und der Verarbeitung der in den pharmazeutischen Zubereitungen verwendeten pankreatinhaltigen Verdauungsenzymgemischen als zweckmäßig zur Stabilisierung der lipolytischen Aktivität während solcher Prozeßschritte des Herstellungs- oder Gewinnungsprozesses, bei denen es zur Inaktivierung der Lipase kommen kann, wie beispielsweise einer Feuchtbehandlung des Enzymgemisches.

In einer wäßrigen Lösung einer ein Verdauungsenzymgemisch enthaltenden pharmazeutischen Zubereitung ist die stabilisierende Wirkung der zugesetzten komplexen Lipide welche ausgewählt sind aus der Gruppe der Phospholipide, Glycolipide und deren Gemische, insbesondere des Lecithins, unter anderem auch abhängig von dem in der Zubereitung herrschenden pH-Wert. Erfindungsgemäß haben sich pH-Werte im Bereich von pH 3,5 - 9,0, bevorzugt im Bereich pH 4,0 - 7,0, besonders bevorzugt im Bereich pH 5,0 - 6,5 als günstig erwiesen.

Zur Erzielung einer merklichen Stabilisierung der lipolytischen Aktivität in den wasserlöslichen pharmazeutischen Zubereitungen der Verdauungsenzymgemische und aus diesen Zubereitungen herstellbaren wäßrigen Lösungen zur Sondenapplikation ist es notwendig, eine gewisse Mindestmenge an komplexen Lipiden welche ausgewählt sind aus der Gruppe der Phospholipide, Glycolipide und deren Gemische, zuzusetzen. Üblicherweise können in den erfindungsgemäßen Zubereitungen verwendete Verdauungsenzymgemische einen in Aktivitätseinheiten ausgedrückten Lipasegehalt von beispielsweise 2.000 bis 200.000 FIP-E/g aufweisen, wenn sie nur Lipasen aus Säugetierpankreassekret enthalten, und von 2.000 bis 500.000 FIP-E/g, wenn sie Lipasen aus Mikroorganismen entweder alleine oder in Kombination mit Lipasen aus Pankreas enthalten. Eine Menge von mindestens 1 Gew.-% komplexer Lipide bezogen auf die eingesetzte Menge an festem pankreatinhaltigem Verdauungsenzymgemisch, beispielsweise eine Menge von 1 bis 10 Gew.-%, ist dann für die Erzielung einer merklichen Stabilisierung geeignet. Eine Zugabe größerer Mengen an komplexen Lipiden ist ebenfalls möglich, verursacht aber keine nennenswerte Verbesserung der Stabilisierung der lipolytischen Aktivität mehr. So eignet sich beispielsweise für die Stabilisierung von Pankreatin oder Pankreatin und zusätzliche Lipase enthaltende Gemische mit komplexen Lipiden, insbesonere Lecithin, ein Zusatz von mindestens 1 Gew.-%, bevorzugt 2 bis 5 Gew.-%, besonders bevorzugt von ca. 3 Gew.-%, Lecithin.

Mit Hilfe der erfindungsgemäßen Verwendung von komplexen Lipiden welche ausgewählt sind aus der Gruppe der Phospholipide, Glycolipide und deren Gemische lassen sich solche aus pharmazeutischen Zubereitungen von pankreatinhaltigen Verdauungsenzymgemischen herstellbare wäßrige Lösungen, die zu einer raschen Abnahme der lipolytischen Aktivität neigen, derart stabilisieren, daß die lipolytische Aktivität der im Gemisch enthaltenen Lipasen über einen längeren Zeitraum nur geringfügig abnimmt. So weist die wäßrige Lösung eines durch Lecithinzusatz stabilisierten F-Pankreatins nach 8-stündiger Inkubationszeit bei Raumtemperatur eine anhand der Methode FIP/ph. Eur. bestimmte lipolytische Restaktivität von 85 % der ursprünglichen Ausgangsaktivität auf. In einer durch komplexe Lipide stabilisierten wäßrigen F-Pankreatinlösung ließen sich selbst nach 24-stündiger Inkubationszeit noch 50 % der anhand der Methode FIP/ph. Eur. bestimmte lipolytischen Anfangsaktivität nachweisen. Dagegen war in einer nicht-stabilisierten Vergleichslösung unter sonst identischen Bedingungen die lipolytische Aktivität nach 8 Stunden auf unter 20 % der Ausgangsaktivität abgesunken.

Die erfindungsgemäße Stabilisierung von wasserlöslichen pharmazeutischen Zubereitungen von Lipasen und Proteasen enthaltenden Verdauungsenzymgemischen gegen eine Abnahme lipolytischer Aktivität in wäßrigem Medium eröffnet die Möglichkeit, Patienten mit derartigen Verdauungsenzymgemischen in Form einer wäßrigen Lösung kontinuierlich durch Einführung in den gastrointestinalen Trakt zu versorgen. Die hierzu eingesetzten wäßrigen Lösungen sollten natürlich keimwachstumsfrei, vorzugsweise sogar steril sein. Keimwachstumsfreie Lösungen können beispielsweise Lösungen sein, worin durch Zusatz von Konservierungsmitteln die Vermehrung selbstvermehrbarer Keime verhindert wird.

Erfindungsgemäß wird ein Kit zur Herstellung von für die kontinuierliche Einführung in den gastrointestinalen Trakt mittels Sonde geeigneten wäßrigen, gegen die Abnahme von lipolytischer Aktivität stabilisierten keimwachstumsfreien Lösungen von Verdauungsenzymgemischen bereitgestellt, dadurch gekennzeichnet, daß es als Bestandteile enthält:

a) eine wasserlösliche feste, keimwachstumsfreie pharmazeutische Zubereitung eines Lipasen und Proteasen enthaltenden Verdauungsenzymgemisches, welche gegebenenfalls eine zur Stabilisierung gegen eine Abnahme der lipolytischen Aktivität der herzustellenden wäßrigen Lösung ausreichende Menge komplexer Lipide welche aus der Gruppe der Phospholipide, Glycolipide und deren Gemische ausgewählt sind enthalten kann,

und

b) eine zur Herstellung der wäßrigen Lösung ausreichende Menge eines keimwachstumsfreien wäßrigen Lösungsmittels, welches neben Wasser physiologisch verträgliche Salze und Hilfsstoffe enthalten kann, und, falls in der unter a) genannten festen pharmazeutischen Zubereitung keine für eine Stabilisierung der herzustellenden wäßrigen Lösung gegen eine Abnahme der lipolytischen Aktivität ausreichende Menge an komplexen Lipiden enthalten ist, zusätzlich eine zur Stabilisierung gegen eine Abnahme der lipolytischen Aktivität der herzustellenden wäßrigen Lösung ausreichende Menge komplexer Lipide enthält.

Insbesondere kann die in dem Kit verwendete feste Zubereitung a) ein gefriergetrocknetes, Lipasen und Proteasen enthaltendes Verdauungsenzymgemisch, welches gegebenenfalls kom- plexe Lipide enthält, darstellen. Bevorzugt handelt es sich bei dem Verdauungsenzymgemisch um ein pankreatinhaltiges Verdauungsenzymgemisch.

Wäßrige Lösungen, welche frei von Keimwachstum sind, können durch Zusatz an sich bekannter Konservierungsmittel, beispielsweise Parabene, erhalten werden. Sterile Lösungen, welche ebenfalls keimwachstumsfreie Lösungen darstellen, können durch an sich bekannte Sterilisationsverfahren, beispielsweise die Sterilfiltration, erhalten werden.

Die in dem Kit enthaltenen Lipide können vorzugsweise als in dem Verdauungsenzymgemisch (Bestandteil a))bereits enthaltene Zusätze vorliegen. Um ein die komplexen Lipide bereits enthaltendes Pulver der Verdauungsenzymgemische herzustellen, kann beispielsweise eine gegebenenfalls keimwachstumsfreie Lösung der komplexen Lipide mit einer gegebenenfalls keimwachstumsfreien Lösung des Verdauungsenzymgemisches vermischt und anschließend nach an sich bekannten Verfahren, beispielsweise der Gefriertrocknung, getrocknet werden. Um hieraus eine per Sonde applizierbare Lösung zu erhalten, muß das die komplexen Lipide sowie das Verdauungsenzymgemisch enthaltende Pulver mit dem ebenfalls im Kit enthaltenen Lösungsmittel, gegebenenfalls unter keimarmen oder keimfreien Bedingungen, vermischt werden. Die komplexen Lipide können aber auch bereits in dem Lösungsmittel (Bestandteil b)), beispielsweise als Kolloid gelöst, vorliegen. Um per Sonde applizierbare Lösungen zu erhalten, muß in diesem Fall die komplexe Lipide enthaltende wäßrige Lösung oder das Kolloid, gegebenenfalls unter sterilen Bedingungen, mit dem Verdauungsenzymgemisch (Bestandteil a)) vermischt werden.

## Ausführungsbeispiele

### 1. Stabilisierung der lipolytischen Aktivität wäßriger Pankreatinlösungen durch Sojalecithin

Um die unterschiedlichen Veränderungen der lipolytischen Aktivität mit und ohne Zusatz komplexer Lipide in wäßrigen Pankreatinzubereitungen zu bestimmen, wurden entsprechende Proben vorbereitet und bei 30 °C inkubiert. Aus den Inkubationsproben bestimmte man die zeitabhängige Änderung der Lipaseaktivitäten nach der Methode der "Fédération Internationale Pharmaceutique/European Pharmacopeia" (im folgenden mit FIP/Ph.Eur. abgekürzt, siehe Lauwers, S. 74 - 82). Bei dieser Standard-Bestimmungsmethode läßt man die auf Lipaseaktivität zu untersuchende Probe unter hydrolytischen Bedingungen auf Olivenöl-Triglyceride einwirken und titriert die freiwerdenden Carbonsäuren mit Natriumhydroxid auf den pH-Wert 9. Die Lipaseaktivität der Probe wird dabei bestimmt durch den Vergleich der Geschwindigkeit, mit der die Probe eine Olivenölemulsion hydrolysiert, mit der Geschwindigkeit, mit welcher eine Suspension eines Pankreas-Referenzpulvers dasselbe Substrat unter denselben Bedingungen hydrolysiert.

### 1.1 Untersuchung der Lipasestabilität ohne Lecithinzusatz

79,35 mg eines wasserlöslichen, gefriergetrockneten F-Pankreatins mit einer lipolytischen Aktivität von 50473 FIP-E/g wurden in 4,0 ml eiskaltem Reinstwasser (Nanopur® der Fa. Barnstead) gelöst, der pH-Wert mit 1N HCl auf 6,2 eingestellt und der Ansatz anschließend mit Reinstwasser auf 5,0 ml aufgefüllt. Aus diesem Ansatz wurde sofort eine Probe zur Bestimmung der lipolytischen Ausgangsaktivität ("Null-Minuten-Probe") entnommen. Der restliche Ansatz wurde bei 30 °C im Wasserbad inkubiert.Zur Probenahme wurde der Ansatz gut durchmischt, die Probe mit einer geeigneten Pipette entnommen und sofort mit eiskaltem Lipaselösungsmittel so verdünnt, daß zur Lipasebestimmung zwischen 0,5 und 1,5 ml Untersuchungslösung mit einer lipolytischen Aktivität von 8 bis 16 FIP-E zur Verfügung standen. Als Lipaselösungsmittel wurde nach FIP/Ph.Eur. eine Lösung von 10,0 g NaCl, 6,06 g Tris-(Hydroxymethyl)-aminomethan (im folgenden als "TRIS" abgekürzt) und 4,9 g Maleinsäureanhydrid in 900 ml Reinstwasser verwendet, deren pH-Wert mit 4 N Natronlauge auf pH 7 eingestellt und die anschließend mit Reinstwasser auf 1.000 ml aufgefüllt wurde.

Um den zeitlichen Verlauf der Lipaseaktivität zu ermitteln, wurden nach 15, 30, 60, 120 und 180 Minuten weitere Proben aus dem thermostatisierten Ansatz entnommen und darin jeweils innerhalb von 30 Minuten die Lipaseaktivität nach der Methode der FIP/Ph.Eur. (Lauwers, S. 78) bestimmt.

Die in der "Null-Minuten-Probe" ermittelte Lipaseaktivität in Einheiten von FIP-E/ml wurde als 100 %-Wert gesetzt und die während der weiteren Inkubationszeit gemessenen Aktivitätswerte werden prozentual auf diesen Wert bezogen. Die Ergebnisse sind in Tabelle 2 aufgeführt.

### 1.2. Untersuchung der Lipasestabilität mit Lecithinzusatz

Zur Herstellung einer Lecithinlösung wurden 100 mg Sojalecithin (Phospholipidgehalt ca 98 % der Firma Roth) bei Raumtemperatur erst mit wenig Reinstwasser angeteigt und dann auf 20,0 ml aufgefüllt. Das Gemisch wurde unter Rühren für ca. 2 Minuten bis zum Erreichen einer homogenen, kolloidalen Lösung mit Ultraschall bestrahlt. Dann wurden 80,0 mg eines wasserlöslichen, gefriergetrockneten F-Pankreatins mit einer lipolytischen Aktivität von 54694 FIP-E/Gramm in 4,0 ml Reinstwasser gelöst und der pH-Wert in der Lösung mit 1N HCl auf 6,2 eingestellt. Man gab 0,4 ml der Lecithinlösung (2,5 % Lecithin, bezogen auf eingesetztes F-Pankreatinpulver) zu und füllte den Ansatz unter guter Durchmischung mit eiskaltem Reinstwasser auf 5,0 ml auf.

Die Probennahme und Bestimmung der lipolytischen Aktivitäten zu den jeweiligen Inkubationszeiten erfolgte wie unter 1.1. beschrieben. Die Ergebnisse sind in Tabelle 2 aufgeführt.

Tabelle 2:

Änderung der lipolyitschen Aktivitäten in wäßrigen Pankreatinlösungen mit und ohne Zusatz von Lecithin

| pH | Zusatz an Lecithin | % lipolytische Aktivität nach t [min] | | | | | |
|---|---|---|---|---|---|---|---|
| | | 0 | 15 | 30 | 60 | 120 | 180 |
| 6,2 | -- | 100 | 69 | 51 | 41 | 26 | 21 |
| 6,2 | 2,5 % | 103 | 93 | 90 | 86 | 80 | 75 |

**2. Stabilisierung der lipolytischen Aktivität wäßriger Pankreatinzubereitungen über einen Zeitraum von 8 Stunden**

In wäßrigen Pankreatinzubereitungen treten in Abhängigkeit von verschiedenen Faktoren wie Temperatur, pH-Wert und proteolytischer Aktivität der enthaltenen Proteasen Verluste in der lipolytischen Aktivität auf. Durch Zusatz von komplexen Lipiden, wie Lecithin, kann die Lipaseaktivität während einer Inkubation über 8 Stunden bei 25 °C deutlich stabilisiert werden. Im folgenden Versuch wurden daher während 8 Stunden die lipolytischen Aktivitäten in wäßrigen Suspensionen und Lösungen von F-Pankreatin jeweils mit und ohne Zusatz komplexer Lipide verglichen.

### 2.1 Herstellung der Inkubationsansätze

Zum Vergleich wurden klare Pankreatinlösungen und Pankreatinsuspensionen aus F-Pankreatin jeweils mit und ohne Lecithinzusatz untersucht.

Es wurden folgende wäßrige Zubereitungen hergestellt:

a) Pankreatinlösung ohne Lecithin
Es wurden 77,5 mg eines wasserlöslichen, gefriergetrockneten F-Pankreatinpulvers wie unter 1.1 beschrieben in eiskaltem Reinstwasser gelöst, wobei der pH-Wert mit 1N HCl auf 6,2 eingestellt wurde.

b) Pankreatinlösung mit Lecithin
81,0 mg eines wasserlöslichen, gefriergetrockneten F-Pankreatinpulvers wurden wie unter 1.2 beschrieben nach Einstellung des pH-Wertes auf 6,2 mit 0,94 ml Lecithinlösung versetzt und mit eiskaltem Reinstwasser auf 5,0 ml aufgefüllt.

c) Pankreatinsuspension ohne Lecithin
2,0 g F-Pankreatin wurden in 100 ml eiskaltem Reinstwasser 30 Minuten lang gerührt. Man erhielt eine trübe Suspension.

d) Pankreatinsuspension mit Lecithin
2,0 g F-Pankreatin wurden mit 100 mg Sojalecithin (Fa. Roth) versetzt und in 100 ml eiskaltem Reinstwasser 30 Minuten lang gerührt. Man erhielt eine trübe Suspension.

### 2.2 Versuchsdurchführung

Aus den unter 2.1 hergestellten eiskalten Lösungen bzw. Suspensionen wurden sofort nach deren Herstellung Proben zur Bestimmung der lipolytischen Ausgangsaktivität entnommen ("Null-Minuten-Proben"). Dann wurde der Rest der Ansätze in Reagenzgläsern 8 Stunden lang bei 25 °C inkubiert. Während dieser Zeit wurden weitere Proben nach 30 Minuten, anschließend stündlich, entnommen. Die Probenahme, Verdünnung und Bestimmung der Lipaseaktivität erfolgte im Prinzip wie unter 1.1 beschrieben, die Versuchstemperatur betrug aber in Abweichung von obiger Vorschrift jetzt 25 °C.

Die Lipaseaktivität (angegeben in FIP-E/ml) der "Null-Minuten-Probe" eines Inkubationsansatzes wurde als 100 %-Wert gesetzt und die während der weiteren Inkubationszeit gemessenen Aktivitätswerte wurden prozentual auf diesen Wert bezogen. Die Ergebnisse sind in den nachfolgenden Tabellen 3 und 4 wiedergegeben.

Tabelle 3:

| Verlauf der Lipasestabilität über 8 Stunden in Pankreatinsuspensionen mit und ohne Lecithinzusatz | | | | | | | | | | | | |
|---|---|---|---|---|---|---|---|---|---|---|---|---|
| **Ansatz** | **% Lecithingehalt** | **pH** | **% Lipolytische Aktivität nach t [h]** | | | | | | | | | |
| | | | **0** | **0,5** | **1** | **2** | **3** | **4** | **5** | **6** | **7** | **8** |
| **a)** | -- | 6,2 | 100 | 72 | 64 | 47 | 36 | 30 | 27 | 24 | 21 | 19 |
| **b)** | 5,8 | 6,2 | 100 | 97 | 98 | 96 | 98 | 94 | 92 | 89 | 85 | 85 |

Tabelle 4:

| Tabelle 4: Verlauf der Lipasestabilität über 8 Stunden in Pankreatinsuspensionen mit und ohne Lecithinzusatz | | | | | | | | | | | | |
|---|---|---|---|---|---|---|---|---|---|---|---|---|
| **Ansatz** | **% Lecithingehalt** | **pH** | **% Lipolytische Aktivität nach t [h]** | **=** | | | | | | | | |
| | | | **0** | **0,5** | **1** | **2** | **3** | **4** | **5** | **6** | **7** | **8** |
| c) | -- | 7,1 | 100 | 72 | 56 | 43 | 34 | 29 | 25 | 21 | 18 | 16 |
| d) | 5 | 7,1 | 100 | 97 | 97 | 92 | 84 | 77 | 72 | 69 | 64 | 60 |

**3. <u>Stabilisierung einer mikrobiellen Lipase gegenüber einer mikrobiellen Protease durch Zusatz komplexer Lipide</u>**

Im folgenden Versuch wurde gezeigt, daß sich auch die Aktivität mikrobieller Lipasen in Anwesenheit aktiver mikrobieller Proteasen durch Zusatz komplexer Lipide stabilisieren ließ. Hierzu wurden drei Untersuchungslösungen hergestellt, welche mikrobielle Lipase, mikrobielle Lipase plus mikrobielle Protease sowie mikrobielle Lipase mit Lecithinzusatz plus mikrobielle Protease enthielten. Anschließend wurden die lipolytischen Aktivitäten in diesen Untersuchungslösungen bestimmt und einander tabellarisch gegenübergestellt.

**3.1 Vorbereitung der Untersuchungslösungen**

Es wurden folgende Lösungen hergestellt:

a) Lipaselösung
245 mg Lipase von Rhizopus oryzae (Lipase 7-AP 15, Amano Pharmaceutical Co., LTD Nagoya, Japan) mit einer Aktivität von 170.000 FIP-E/g wurden in 50 ml eiskalter, 1%-iger Natriumchloridlösung gelöst.

b) Proteaselösung
390 mg Aspergillus-Protease (Prozyme 6; Amano Pharmaceutical Co., LTD Nagoya, Japan) mit einer Aktivität von 7.600 FIP-E/g wurden in 25 ml eiskalter, 1%-iger Natriumchloridlösung gelöst.

c) Lecithinlösung
1 g Lecithin (Soja-Reinlecithin Phospholipidgehalt ca. 98 %, der Firma Roth) wurde in 40 ml Reinstwasser ("Nanopure®" der Firma Barnstead) aufgenommen und unter Umschütteln ca. 2 Minuten lang mit Ultraschall zu einem Kolloid gelöst.
Aus diesen Lösungen wurden im Eisbad die folgenden Inkubationslösungen im Gesamtvolumen von 5 ml hergestellt:

Tabelle 5:

| Inkubationslösungen zur Bestimmung der Rhizopus-Lipase-Aktivität | | | | |
|---|---|---|---|---|
| **Inkubationslösung** | **Lipaselösung** | **Proteaselösung** | **Lecithinlösung** | **NaCl-Lösung** |
| **I** | 3 ml | -- | -- | 2 ml |
| **II** | 3 ml | 1 ml | -- | 1 ml |
| **III** | 3 ml | 1 ml | 1 ml | -- |

Der pH-Wert aller Inkubationslösungen betrug nach der Herstellung 6,5 bei 37 °C.

**3.2 Versuchsdurchführung**

Aus den eiskalten Inkubationslösungen I, II und III wurden sofort nach deren Herstellung Proben zur Bestimmung der Ausgangsaktivitäten ("Null-Minuten-Proben") entnommen, der Rest der drei Ansätze wurde in Reagenzgläsern bei 37 °C im Wasserbad inkubiert.

Zur Probenahme wurde der Ansatz gut durchmischt, die Probe mit einer geeigneten Pipette entnommen und sofort mit eiskaltem Lipaselösungsmittel wie unter 1.1 beschrieben verdünnt. Diesen Probenlösungen wurden jeweils bestimmte Mengen entnommen (in Tabelle 6 mit "X" gekennzeichnet) und mit 1 %-iger Natriumchloridlösung auf 5 ml verdünnt. Von diesen Untersuchungslösungen wurden jeweils 0,5 ml im Lipase-Aktivitätstest eingesetzt.

Tabelle 6:

| Entnommene Probenmengen zur Herstellung der Untersuchungslösungen | | | |
|---|---|---|---|
| **Untersuchungslösung** | **X µl Probenlösung entnommen nach t =** | | |
| | **0 min** | **30 min** | **60 min** |
| **I** | 150 | -- | 160 |
| **II** | 160 | 500 | 1000 |
| **III** | 160 | 250 | 250 |

**3.3 Bestimmung der Rhizopus-Lipase-Aktivität**

Die katalytische Aktivität der Rhizopus-Lipase wurde durch Bestimmung der Menge an freien Fettsäuren gemessen, die über einen definierten Zeitraum bei pH 7,0 und 37 °C aus einer Olivenöl-Emulsion in Gegenwart von 0,025 % Natriumtaurocholat gebildet wurden. Um zu gewährleisten, daß alle Fettsäuren erfaßt wurden, erfolgte anschließend eine Titration bis pH 9,0. Eine Blindwertbestimmung durch Titration der Substrat-Emulsion diente der Erfassung von titrierbaren Substanzen, die nicht durch die Lipase-Aktivität entstanden sind.

Die Rhizopus-Lipase-Aktivität einer Untersuchungssubstanz wurde bestimmt durch den Vergleich der Geschwindigkeit, mit der eine Suspension der Substanz eine Olivenöl-Emulsion hydrolysierte, mit der Geschwindigkeit, mit der eine Suspension eines Rhizopus-Lipase-Referenzstandards die gleiche Olivenöl-Emulsion unter denselben Bedingungen hydrolysierte.

<u>**Reagenzien**</u>

<u>1. Wasser:</u>

Es wurde Reinstwasser "Nanopure®" der Firma Barnstead verwendet. Es wird im folgenden als "Reinstwasser" bezeichnet.

<u>2. Arabisch Gummi-Lösung:</u>

110 g Arabisches Gummi und 12,5 g Calciumchlorid wurden unter Rühren (ca. 3 Stunden) in Reinstwasser gelöst, auf 1.000 ml aufgefüllt und zentrifugiert. Die Lösung wurde in 250 ml Plastikgefäße abgefüllt und bei -20 °C gelagert.

Arabisches Gummi (Acaciae-Gummi, DAB 10/Ph.Eur.), Calciumchlorid p.a.

<u>3. Substratemulsion:</u>

130 ml Olivenöl und 400 ml Arabisch Gummi-Lösung (2) wurden 15 Minuten lang in einem geeigneten Rührgerät mit hoher Umdrehungszahl emulgiert. Die Temperatur wurde unter 30 °C gehalten. Mindestens 90 % der Tröpfchen in der Emulsion besaßen einen Durchmesser von weniger als 3 µm und keines war größer als 10 µm. Die Emulsion wurde täglich frisch hergestellt.
Olivenöl (im Kühlschrank gelagert), DAB-Qualität.

<u>4. Natriumtaurocholat-Lösung 0,5 % (m/V):</u>

0,5 g Natriumtaurocholat (Lipase activating mixture, Gemisch von konjugierten Gallensäuren, u. a. Natriumtaurocholat) wurde mit Reinstwasser zu 100,0 ml gelöst. Die Lösung wurde täglich frisch angesetzt.
Natriumtaurocholat (Lipase activating mixture), FIP.

5. Natriumchloridlösung 1 % (m/V) in Reinstwasser:

6. Pufferlösung pH 4,5:

2 g Natriumchlorid und 9,2 g Natriumdihydrogenphosphat wurden in ca. 950 ml Reinstwasser gelöst, mit Salzsäure auf pH 4,5 eingestellt und mit Reinstwasser auf 1.000 ml aufgefüllt. Natriumchlorid p. a., Natriumdihydrogenphosphat, $NaH_2PO_4$ X $H_2O$.

7. Natronlauge 0,1 N

**Referenzsuspension:**

Der Rhizopus-Lipase-Referenzstandard wurde in Pufferlösung (6) gerührt und mit Natriumchloridlösung (5) soweit verdünnt, bis die Enzymlösung 12 bis 18 Einheiten der Rhizopus-Li-paseaktivität FIP-E/ml enthielt. Bei einem Standard mit 55.000 FIP-E pro 1 g löste man 63 mg in 20 ml Pufferlösung (6) innerhalb von 15 Minuten im Eisbad. 10 ml dieser Lösung verdünnte man mit Natriumchloridlösung (5) zu 100 ml im Eisbad. 0,5 ml dieser Lösung wurden im Test eingesetzt.

Rhizopus-Lipase-Referenzstandard FIP (Fungi-Lipase, FIP-Standard).

**Untersuchungslösungen:**

Es wurden die Untersuchungslösungen I, II und III verwendet.

**Versuchsdurchführung:**

Am Autotitrator eines Titriersystems "pH-Stat" der Firma Radiometer wurde der Endpunkt für die pH-Stat-Titration auf pH 7,0 eingestellt. In das Reaktionsgefäß wurden gegeben:

| | |
|---|---|
| 12,0 ml | Olivenölemulsion, FIP (3) |
| 6,5 ml | Reinstwasser (1) |
| 1,0 ml | Natriumtaurocholat-Lösung (4) |

Die Mischung wurde auf 37,0 °C gebracht. Der pH-Wert wurde mit 0,1 N Natronlauge (7) auf pH 7,0 eingestellt. Danach wurde die Bürette auf "0" gestellt.

Die Reaktion wurde durch Zugabe von 0,5 ml Referenzsuspension gestartet, wenn der Referenzwert gemessen werden sollte, oder 0,5 ml Untersuchungslösung, wenn die Lipaseaktivität in der Untersuchungslösung gemessen werden sollte. Nach 10 Minuten wurde der Endpunkt für die pH-Stat-Titration auf pH 9,0 eingestellt. Wenn der pH-Wert 9,0 erreicht war (dieser Vorgang dauerte weniger als 30 Sekunden), wurde die Titration abgebrochen und der Verbrauch an 0,1 N Natronlauge abgelesen.

Zur Ermittlung der Blindwerte wurde für die Referenzsuspension und die Untersuchungslösung der Endpunkt der Titration am Titrator sofort auf pH 9,0 eingestellt. Nach manueller Einstellung des pH-Wertes im Reaktionsansatz auf pH 7,0 und nach Zugabe von 0,5 ml Referenzsuspension oder Untersuchungslösung wurde sofort auf pH 9,0 titriert. Aus dem abgelesenen Verbrauch an 0,1 N Natronlauge wurde die Rhizopus-Lipaseaktivität in FIP-E/ml Untersuchungslösung berechnet.

Die in den Untersuchungslösungen I, II und III ermittelten Anfangs-Lipseaktivitäten in Einheiten von FIP-E/ml werden als 100 %-Werte gesetzt. Die während der folgenden Inkubationszeit von 1 Stunde gemessenen Aktivitätswerte wurden dann jeweils prozentual auf diese Ausgangswerte bezogen und in Tabelle 7 aufgeführt.

Tabelle 7:

| Stabilisierung der Aktivität von Rhizopus-Lipase durch Sojalecithin | | | |
|---|---|---|---|
| **Untersuchungslösung** | **% lipolytische Aktivität nach t =** | | |
| | **0 min** | **30 min** | **60 min** |
| I Lipaselösung | 100 | 100 | 95 |
| II Lipaselösung + Protease | 100 | 17 | 6 |

Tabelle 7: (fortgesetzt)

| Stabilisierung der Aktivität von Rhizopus-Lipase durch Sojalecithin | | | |
|---|---|---|---|
| **Untersuchungslösung** | **% lipolytische Aktivität nach t =** | | |
| | **0 min** | **30 min** | **60 min** |
| III Lipaselösung mit Lecithin + Protease | 100 | 94 | 84 |

**4. <u>Beispiel einer pharmazeutischen Zubereitung</u>**

A) Man nimmt 20,0 g F-Pankreatin in 400 ml Wasser auf und stellt den pH-Wert durch Zugabe von 1 N HCl rasch auf 6,2 ein. Bei diesen Bedingungen extrahiert man 1 Stunde lang bei 4 °C. Man zentrifugiert den klaren, den Pankreatin-Extrakt enthaltenden Überstand bei 53.000g und filtriert diesen anschließend durch Filter von 2µm Porengröße steril.

B) 1,0 g Sojalecithin (98 % an Phosphatidylcholin) werden in 50 ml Wasser gelöst und in einer abgeschmolzenen Glasampulle 45 Minuten lang bei 140 °C sterilisiert.

C) 400 ml des unter A) hergestellten Pankreatinextraktes werden mit 25 ml der unter B) hergestellten Lecithinlösung in der Kälte und unter sterilen Bedingungen gemischt. Das Gemisch wird gefriergetrocknet, wobei 16 g eines Pulvers erhalten werden, welches man unter sterilen Bedingungen in Portionen zu 2,5 g in Infusionsflaschen von 100 ml abfüllt.

**5. <u>Beispiele für Kits zur Pharmazeutischen Anwendung</u>**

**5.1. Kit 1**

A) 40,0 g F-Pankreatin werden in 400 ml Wasser aufgenommen und der pH-Wert wird durch Zugabe von 1 N HCl rasch auf 6,2 eingestellt. Bei diesen Bedingungen wird 1 Stunde lang bei 4 °C extrahiert. Man zentrifugiert den klaren, den Pankreatin-Extrakt enthaltenden Überstand bei 53.000g und filtriert diesen anschließend durch Filter von 2µm Porengröße steril. Der Extrakt wird unter sterilen Bedingungen in Portionen von 25 ml in 100 ml Infusionsflaschen abgefüllt und gefriergetrocknet.

B) 2,0 g Sojalecithin (98 % an Phosphatidylcholin) werden in 20 ml Wasser gelöst und mit Ultraschall homogenisiert. Diese kolloidale Lösung wird in einer abgeschmolzenen Glasampulle 45 Minuten lang bei 140 °C sterilisiert. Man entnimmt hiervon eine Portion von 12,5 ml, welche man mit 1600 ml sterilisiertem Wasser vermischt und in Portionen von 100 ml in Infusionsflaschen abfüllt.

Zur Anwendung wird der Inhalt einer Flasche des unter A) erhaltenen Feststoffes in dem Inhalt einer Flasche der unter B) erhaltenen Lösung gelöst.

**5.2. Kit 2**

A) 40,0 g F-Pankreatin werden in 800 ml Wasser aufgenommen und der pH-Wert wird durch Zugabe von 1 N HCl rasch auf 6,2 eingestellt. Bei diesen Bedingungen wird 1 Stunde lang bei 4 °C extrahiert. Man zentrifugiert den klaren, den Pankreatin-Extrakt enthaltenden Überstand bei 53.000g und verwirft den Niederschlag.

B) 32,0 g Mannit werden in 200 ml Wasser gelöst, mit 800 ml des unter A) erhaltenen Pankreatinextraktes vermischt und die vereinigten Lösungen werden sterilfiltriert.

C) 5,0 g Sojalecithin (98 % an Phosphatidylcholin) werden in 50 ml Wasser gelöst und mit Ultraschall homogenisiert. Diese kolloidale Lösung wird in einer abgeschmolzenen Glasampulle 45 Minuten lang bei 140 °C sterilisiert.

D) 1000 ml des unter B) erhaltenen Pankreatinextraktes werden unter sterilen Bedingungen mit 16 ml der unter C) erhaltenen Lecithinlösung gemischt und gefriergetrocknet. Das erhaltene Pulver wird in Portionen zu 10,0 g in Flaschen von 200 ml steril abgefüllt.

E) Entionisiertes und sterilfiltriertes Wasser wird in Flaschen zu 200 ml unter sterilen Bedingungen abgefüllt.

Zur Anwendung wird der Inhalt einer Flasche des unter D) erhaltenen Pulvers in dem Inhalt einer Flasche des unter E) abgefüllten Wassers gelöst.

**Patentansprüche**

1. Verwendung von komplexen Lipiden als gegen eine Abnahme von lipolytischer Aktivität unter Feuchtigkeitseinfluß stabilisierende Zusätze in wasserlöslichen pharmazeutischen Zubereitungen von Lipasen und Proteasen enthaltenden Verdauungsenzymgemischen, welche für die Herstellung von wäßrigen Lösungen zur kontinuierlichen Einführung in den gastrointestinalen Trakt mittels Sonden geeignet sind, wobei die komplexen Lipide ausgewählt sind aus der Gruppe der Phospholipide, Glycolipide und deren Gemische.

2. Verwendung von komplexen Lipiden gemäß Anspruch 1, wobei die Lipasen und Proteasen enthaltenden Verdauungsenzymgemische pankreatinhaltige Verdauungsenzymgemische sind.

3. Verwendung von komplexen Lipiden gemäß Anspruch 2, **dadurch gekennzeichnet, daß** sie als stabilisierende Zusätze zu pharmazeutischen Zubereitungen von pankreatinhaltigen Verdauungsenzymgemischen, welche neben Pankreatin zusätzliche Lipasen, ausgewählt aus der Gruppe bestehend aus Pflanzenlipasen, bakteriellen Lipasen und Lipasen aus Pilzkulturen, enthalten, eingesetzt werden.

4. Verwendung von komplexen Lipiden gemäß einem der vorstehenden Ansprüche, **dadurch gekennzeichnet, daß** als komplexe Lipide Phospholipide oder diese enthaltende Lipidgemische verwendet werden.

5. Verwendung von komplexen Lipiden gemäß Anspruch 4, **dadurch gekennzeichnet, daß** als Phospholipide Salze von Anionen der allgemeinen Formel I

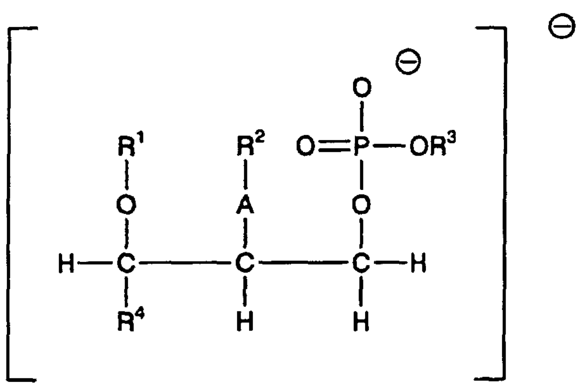

I

worin

$R^1$ Wasserstoff oder einen Alkanoylrest mit 10 - 25 Kohlenstoffatomen, dessen Kohlenwasserstoffrest gegebenenfalls 1 - 4 Doppelbindungen enthalten kann, bedeutet,

$R^2$ einen Alkanoylrest mit 10 - 25 Kohlenstoffatomen, dessen Kohlenstoffrest gegebenenfalls 1 - 4 Doppelbindungen enthalten kann, bedeutet, oder, wenn $R^1$ nicht für Wasserstoff steht, auch Wasserstoff bedeuten kann,

$R^3$ Wasserstoff, eine niedere Alkylgruppe, welche durch Amino, niederes Trialkylammonium, eine an ein eine Aminofunktion tragendes Kohlenstoffatom gebundene Carboxylgruppe oder eine durch Hydroxy substituierte Cycloalkylgruppe substituiert sein kann, bedeutet,

$R^4$ Wasserstoff oder eine Kohlenwasserstoffkette mit 10 - 25 Kohlenstoffatomen, die gegebenenfalls 1 - 4 Doppelbindungen enthalten kann, bedeutet,

A für Sauerstoff oder NH steht,

mit physiologisch verträglichen Kationen, oder deren Gemische verwendet werden.

**6.** Verwendung von komplexen Lipiden gemäß Anspruch 5, **dadurch gekennzeichnet, daß** in den Phospholipiden der allgemeinen Formel I der Rest $R^3$ ausgewählt ist aus der Gruppe bestehend aus Wasserstoff, Aminoethyl, Cholyl, Seryl, Glyceryl oder myo-Inosityl.

**7.** Verwendung von komplexen Lipiden gemäß Anspruch 6, **dadurch gekennzeichnet, daß** Phospholipide aus der Gruppe bestehend aus Phosphatidsäure, Phosphatidylcholin, Phosphatidylserin, Phosphatidylethanolamin, Phosphatidylinosit und Sphingomyelin oder diese enthaltende Lipidgemische verwendet werden.

**8.** Verwendung von komplexen Lipiden gemäß Anspruch 1, **dadurch gekennzeichnet, daß** als komplexe Lipide Lecithine aus Sojabohnen, Raps, Mais, Sonnenblumen, Erdnüssen, Hühnerei oder Mikroorganismen verwendet werden.

**9.** Verwendung von komplexen Lipiden gemäß Anspruch 8, **dadurch gekennzeichnet, daß** als komplexe Lipide Lecithine aus Sojabohnen verwendet werden.

**10.** Verwendung von komplexen Lipiden gemäß einem der Ansprüche 2 bis 9, **dadurch gekennzeichnet, daß** die Lipide dem in den pharmazeutischen Zubereitungen eingesetzten Pankreatin bereits bei dessen Gewinnung oder Verarbeitung zur Stabilisierung gegen eine Abnahme der lipolytischen Aktivität während mit einer Feuchtbehandlung verbundener Prozeßschritte zugesetzt werden.

**11.** Wasserlösliche pharmazeutische Zubereitungen von Lipasen und Proteasen enthaltenden Verdauungsenzymgemischen, welche zur Herstellung von wäßrigen Lösungen zur kontinuierlichen Einführung in den gastrointestinalen Trakt mittels Sondenapplikation geeignet sind, **dadurch gekennzeichnet, daß** sie eine zur Stabilisierung gegen eine Abnahme von lipolytischer Aktivität unter Feuchtigkeitseinfluß ausreichende Menge komplexer Lipide gemäß einem der Ansprüche 1 bis 9 enthalten.

**12.** Pharmazeutische Zubereitungen gemäß Anspruch 11, **dadurch gekennzeichnet, daß** es sich bei den Lipasen und Proteasen enthaltenden Verdauungsenzymgemischen um pankreatinhaltige Verdauungsenzymgemische handelt.

**13.** Pharmazeutische Zubereitungen gemäß Anspruch 12, **dadurch gekennzeichnet, daß** die Verdauungsenzymgemische neben Pankreatin zusätzliche Lipasen ausgewählt aus der Gruppe bestehend aus Pflanzenlipasen, bakteriellen Lipasen und Lipasen aus Pilzkulturen enthalten.

**14.** Pharmazeutische Zubereitungen gemäß einem der Ansprüche 11 bis 13, **dadurch gekennzeichnet, daß** sie eine solche Menge komplexer Lipide enthalten, daß von wäßrigen Lösungen der Zubereitungen die lipolytische Aktivität derart stabilisiert wird, daß die lipolytische Ausgangsaktivität einer wäßrigen Lösung über einen Zeitraum von 8 Stunden um höchstens 20 % abnimmt, wobei die lipolytische Aktivität anhand der Methode gemäß FIP/Ph.Eur. bestimmt wird.

**15.** Pharmazeutische Zubereitungen gemäß Anspruch 14, **dadurch gekennzeichnet, daß** sie eine solche Menge komplexer Lipide enthalten, daß die lipolytische Ausgangsaktivität von wäßrigen Lösungen der Zubereitungen über einen Zeitraum von 24 Stunden um höchstens 50 % abnimmt, wobei die lipolytische Aktivität anhand der Methode gemäß FIP/Ph.Eur. bestimmt wird.

**16.** Pharmazeutische Zubereitungen gemäß einem der Ansprüche 11 bis 13, **dadurch gekennzeichnet, daß** sie komplexe Lipide zur Stabilisierung gegen eine Abnahme von lipolytischer Aktivität in einer Konzentration von 1 bis 10 Gew.-%, vorzugsweise von 2 bis 5 Gew.-%, bezogen auf das Verdauungsenzymgemisch enthalten.

**17.** Pharmazeutische Zubereitungen gemäß einem der Ansprüche 11 bis 16, **dadurch gekennzeichnet, daß** das darin enthaltene Pankreatin autolytisch vorbehandeltes Pankreatin ist.

**18.** Kit zur Herstellung von für die kontinuierliche Einführung in den gastrointestinalen Trakt mittels Sonden geeigneten, gegen die Abnahme von lipolytischer Aktivität stabilisierten keimwachstumsfreien wäßrigen Lösungen von Verdauungsenzymgemischen, **dadurch gekennzeichnet, daß** es als Bestandteile enthält:

a) eine wasserlösliche feste, keimwachstumsfreie pharmazeutische Zubereitung eines Lipasen und Proteasen enthaltenden Verdauungsenzymgemisches, welche gegebenenfalls eine zur Stabilisierung gegen eine Abnahme der lipolytischen Aktivität der herzustellenden wäßrigen Lösung ausreichende Menge komplexer Lipide gemäß einem der Ansprüche 1 bis 9 enthalten kann,

und

b) eine zur Herstellung der wäßrigen Lösung ausreichende Menge eines keimwachstumsfreien wäßrigen Lösungsmittels, welches neben Wasser physiologisch verträgliche Salze und Hilfsstoffe enthalten kann, und, falls in der unter a) genannten festen pharmazeutischen Zubereitung keine für eine Stabilisierung der herzustellenden wäßrigen Lösung gegen eine Abnahme der lipolytischen Aktivität ausreichende Menge an komplexen Lipiden enthalten ist, zusätzlich eine zur Stabilisierung gegen eine Abnahme der lipolytischen Aktivität der herzustellenden wäßrigen Lösung ausreichende Menge komplexer Lipide gemäß einem der Ansprüche 1 bis 9 enthält.

**19.** Kit gemäß Anspruch 18, **dadurch gekennzeichnet, daß** die feste Zubereitung a) ein gefriergetrocknetes, Lipasen und Proteasen enthaltendes Verdauungsenzymgemisch, welches gegebenenfalls komplexe Lipide enthält, darstellt.

**20.** Kit gemäß Anspruch 19, **dadurch gekennzeichnet, daß** das gefriergetrocknete Verdauungsenzymgemisch Pankreatin enthält.

**Claims**

**1.** Use of complex lipids as additives stabilising against a decrease in lipolytic activity under the influence of moisture in water-soluble pharmaceutical preparations of digestive enzyme mixtures which contain lipases and proteases and which are suitable for preparing aqueous solutions for continuous introduction into the gastrointestinal tract by means of tubes, the complex lipids being selected from the group consisting of phospholipids, glycolipids and mixtures thereof.

**2.** Use of complex lipids according to Claim 1, wherein the digestive enzyme mixtures containing lipases and proteases are pancreatin-containing digestive' enzyme mixtures.

**3.** Use of complex lipids according to Claim 2, **characterised in that** they are employed as stabilising additives to pharmaceutical preparations of pancreatin-containing digestive enzyme mixtures which contain, besides pancreatin, additional lipases selected from the group consisting of plant lipases, bacterial lipases and lipases from fungus cultures.

**4.** Use of complex lipids according to any one of the preceding claims, **characterised in that** phospholipids or lipid mixtures containing the latter are used as complex lipids.

**5.** Use of complex lipids according to Claim 4, **characterised in that** salts of anions of the general formula I

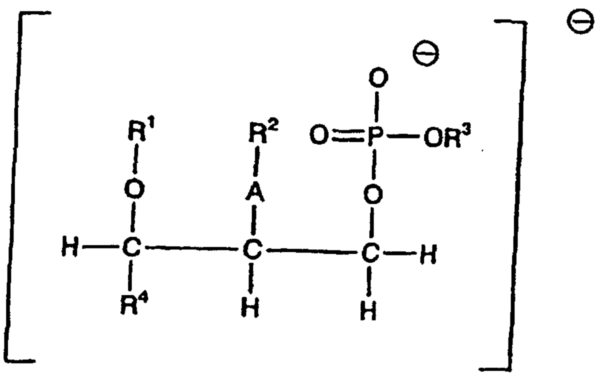

I

in which

$R^1$ denotes hydrogen or an alkanoyl radical with 10-25 carbon atoms, whose hydrocarbon radical may optionally contain 1-4 double bonds,

$R^2$ denotes an alkanoyl radical with 10-25 carbon atoms, whose carbon radical may optionally contain 1-4 double bonds, or, if $R^1$ does not represent hydrogen, can also denote hydrogen,

$R^3$ denotes hydrogen, a lower alkyl group which can be substituted by amino, lower trialkylammonium, a carboxyl group bonded to a carbon atom carrying an amino functionality, or a hydroxyl-substituted cycloalkyl group,

$R^4$ denotes hydrogen or a hydrocarbon chain with 10-25 carbon atoms, which may optionally contain 1-4 double bonds,

A represents oxygen or NH,

with physiologically tolerated cations, or mixtures thereof, are used as phospholipids.

**6.** Use of complex lipids according to Claim 5, **characterised in that** the radical $R^3$ in the phospholipids of the general formula I is selected from the group consisting of hydrogen, aminoethyl, cholyl, seryl, glyceryl or myo-inosityl.

**7.** Use of complex lipids according to Claim 6, **characterised in that** phospholipids from the group consisting of phosphatidic acid, phosphatidylcholine, phosphatidylserine, phosphatidylethanolamine, phosphatidylinositol and sphingomyelin or lipid mixtures containing the latter are used.

**8.** Use of complex lipids according to Claim 1, **characterised in that** lecithins from soya beans, rape, maize, sunflowers, groundnuts, chicken egg or microorganisms are used as complex lipids.

**9.** Use of complex lipids according to Claim 8, **characterised in that** lecithins from soya beans are used as complex lipids.

**10.** Use of complex lipids according to any of Claims 2 to 9, **characterised in that** the lipids are added to the pancreatin employed in the pharmaceutical preparations even while it is being isolated or processed to stabilise against a decrease in the lipolytic activity during process steps associated with wet treatment.

**11.** Water-soluble pharmaceutical preparations of digestive enzyme mixtures containing lipases and proteases, which are suitable for preparing aqueous solutions for continuous introduction into the gastrointestinal tract by tube administration, **characterised in that** they contain a sufficient amount of complex lipids according to any of Claims 1 to 9 for stabilisation against a decrease in lipolytic activity under the influence of moisture.

**12.** Pharmaceutical preparations according to Claim 11, **characterised in that** the digestive enzyme mixtures containing lipases and proteases are pancreatin-containing digestive enzyme mixtures.

**13.** Pharmaceutical preparations according to Claim 12, **characterised in that** the digestive enzyme mixtures contain, besides pancreatin, additional lipases selected from the group consisting of plant lipases, bacterial lipases and lipases from fungus cultures.

**14.** Pharmaceutical preparations according to any of Claims 11 to 13, **characterised in that** they contain an amount of complex lipids such that the lipolytic activity of aqueous solutions of the preparations is stabilised so that the initial lipolytic activity of an aqueous solution decreases by not more than 20% over a period of 8 hours, the lipolytic activity being determined by the method in accordance with FIP/Ph.Eur.

**15.** Pharmaceutical preparations according to Claim 14, **characterised in that** they contain an amount of complex lipids such that the initial lipolytic activity of aqueous solutions of the preparations decreases by not more than 50% over a period of 24 hours, the lipolytic activity being determined by the method in accordance with FIP/Ph.Eur.

**16.** Pharmaceutical preparations according to any of Claims 11 to 13, **characterised in that** they contain complex lipids for stabilising against a decrease in lipolytic activity in a concentration of from 1 to 10% by weight, preferably from 2 to 5% by weight, based on the digestive enzyme mixture.

**17.** Pharmaceutical preparations according to any of Claims 11 to 16, **characterised in that** the pancreatin contained therein is autolytically pretreated pancreatin.

**18.** Kit for preparing aqueous solutions, which are suitable for continuous introduction into the gastrointestinal tract by means of tubes, are stabilised against the decrease in lipolytic activity and are free of microbe growth, of digestive enzyme mixtures, **characterised in that** it contains as components:

a) a water-soluble solid pharmaceutical preparation, which is free of microbe growth, of a digestive enzyme mixture containing lipases and proteases, which may optionally contain an amount of complex lipids according to any of Claims 1 to 9 which is sufficient for stabilisation against a decrease in the lipolytic activity of the aqueous solution to be prepared,

and

b) an amount, sufficient for preparing the aqueous solution, of an aqueous solvent which is free of microbe growth and which, besides water, may contain physiologically tolerated salts and ancillary substances and, if the solid pharmaceutical preparation mentioned under a) does not contain a sufficient amount of complex lipids for stabilisation of the aqueous solution to be prepared against a decrease in the lipolytic activity, additionally contains a sufficient amount of complex lipids according to any of Claims 1 to 9 for stabilisation against a decrease in the lipolytic activity of the aqueous solution to be prepared.

**19.** Kit according to Claim 18, **characterised in that** the solid preparation a) represents a freeze-dried digestive enzyme mixture which contains lipases and proteases and which optionally contains complex lipids.

**20.** Kit according to Claim 19, **characterised in that** the freeze-dried digestive enzyme mixture contains pancreatin.

## Revendications

**1.** Utilisation de lipides complexes en tant qu'additifs de stabilisation contre une réduction de l'activité lipolytique sous influence de l'humidité dans des préparations pharmaceutiques hydrosolubles de mélanges d'enzymes digestives contenant des lipases et des protéases, et qui sont appropriés pour la préparation de solutions aqueuses destinées à l'introduction en continu dans le tractus gastro-intestinal au moyen de sondes, les lipides complexes étant sélectionnés dans le groupe composé des phospholipides, des glycolipides et de leurs mélanges.

**2.** Utilisation de lipides complexes selon la revendication 1, dans laquelle les mélanges d'enzymes digestives contenant des lipases et des protéases sont des mélanges d'enzymes digestives contenant de la pancréatine.

**3.** Utilisation de lipides complexes selon la revendication 2, **caractérisée en ce qu'**ils sont utilisés en tant qu'additifs de stabilisation dans des préparations pharmaceutiques de mélanges d'enzymes digestives contenant de la pancréatine, et qui, outre de la pancréatine, contiennent des lipases supplémentaires sélectionnées dans le groupe

composé de lipases végétales, de lipases bactériennes et de lipases issues de cultures fongiques.

**4.** Utilisation de lipides complexes selon l'une des revendications précédentes, **caractérisée en ce que** l'on utilise comme lipides complexes des phospholipides ou des mélanges de lipides contenant ceux-ci.

**5.** Utilisation de lipides complexes selon la revendication 4, **caractérisée en ce que** l'on utilise comme phospholipides des sels d'anions de formule générale I

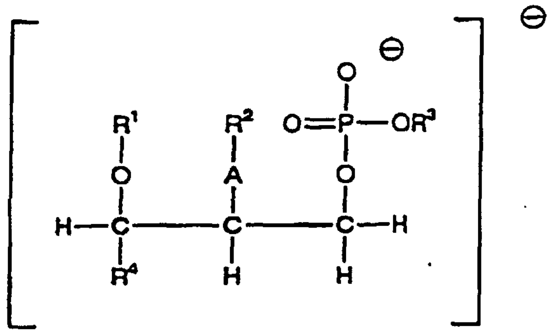

I

dans laquelle

- $R^1$ est un hydrogène ou un radical alcanoyle comportant 10 à 25 atomes de carbone, et dont le radical hydrocarboné peut contenir éventuellement 1 à 4 doubles liaisons,
- $R^2$ est un radical alcanoyle comportant 10 à 25 atomes de carbone, et dont le radical hydrocarboné peut éventuellement comporter 1 à 4 doubles liaisons, ou, lorsque $R^1$ n'est pas un hydrogène, $R^2$ peut aussi représenter un hydrogène,
- $R^3$ est un hydrogène, un groupe alkyle inférieur qui peut être substitué par un amino, un trialkylammonium inférieur, un groupe carboxyle lié à un atome de carbone qui porte une fonction amino, ou un groupe cycloalkyle substitué par un hydroxy,
- $R^4$ est un hydrogène ou une chaîne hydrocarbonée comportant 10 à 25 atomes de carbone, qui peut éventuellement contenir 1 à 4 doubles liaisons,
- A est un oxygène ou NH,

avec des cations physiologiquement compatibles, ou leurs mélanges.

**6.** Utilisation de lipides complexes selon la revendication 5, **caractérisée en ce que**, dans les phospholipides de formule générale I, le radical $R^3$ est sélectionné dans le groupe composé d'un hydrogène, d'un aminoéthyle, d'un cholyle, d'un séryle, d'un glycéryle et d'un myoinosityle.

**7.** Utilisation de lipides complexes selon la revendication 6, **caractérisée en ce que** l'on utilise des phospholipides issus du groupe composé de l'acide phosphatidique, de la phosphatidylcholine, de la phosphatidylsérine, de la phosphatidyléthanolamine, du phosphatidylinositol et de la sphingomyéline, ou de mélanges de lipides les contenant.

**8.** Utilisation de lipides complexes selon la revendication 1, **caractérisée en ce que** l'on utilise comme lipides complexes des lécithines issues de graines de soja, de colza, de maïs, de tournesol, d'arachides, d'oeufs de poules ou de micro-organismes.

**9.** Utilisation de lipides complexes selon la revendication 8, **caractérisée en ce que** l'on utilise comme lipides complexes des lécithines issues de graines de soja.

**10.** Utilisation de lipides complexes selon l'une des revendications 2 à 9, **caractérisée en ce que** les lipides sont ajoutés à la pancréatine utilisée dans les préparations pharmaceutiques dès son obtention ou son traitement à des fins de stabilisation contre une réduction de l'activité lipolytique pendant les étapes de procédé associées à un traitement humide.

**11.** Préparations pharmaceutiques hydrosolubles de mélanges d'enzymes digestives contenant des lipases et des protéases, et qui sont appropriées pour la préparation de solutions aqueuses destinées à une introduction en continu dans le tractus gastro-intestinal au moyen d'une application par sonde, **caractérisées en ce qu'**elles contiennent une quantité de lipides complexes selon l'une des revendications 1 à 9 suffisante pour une stabilisation contre une réduction de l'activité lipolytique sous influence de l'humidité.

**12.** Préparations pharmaceutiques selon la revendication 11, **caractérisées en ce qu'**il s'agit concernant les mélanges d'enzymes digestives contenant des lipases et des protéases de mélanges d'enzymes digestives contenant de la pancréatine.

**13.** Préparations pharmaceutiques selon la revendication 12, **caractérisées en ce que** les mélanges d'enzymes digestives, outre de la pancréatine, contiennent des lipases supplémentaires sélectionnées dans le groupe composé de lipases végétales, de lipases bactériennes et de lipases issues de cultures fongiques.

**14.** Préparations pharmaceutiques selon l'une des revendications 11 à 13, **caractérisées en ce qu'**elles contiennent une quantité de lipides complexes telle qu'à partir de solutions aqueuses des préparations, l'activité lipolytique est stabilisée de façon telle que l'activité lipolytique de départ d'une solution aqueuse est réduite d'au plus 20 % sur une durée de 8 heures, l'activité lipolytique étant déterminée selon la méthode conforme à FIP/Ph. Eur.

**15.** Préparations pharmaceutiques selon la revendication 14, **caractérisées en ce qu'**elles contiennent une quantité de lipides complexes telle que l'activité lipolytique de départ de solutions aqueuses des préparations est réduite d'au plus 50 % sur une durée de 24 heures, l'activité lipolytique étant déterminée selon la méthode conforme à FIP/Ph. Eur.

**16.** Préparations pharmaceutiques selon l'une des revendications 11 à 13, **caractérisées en ce qu'**elles contiennent des lipides complexes destinés à une stabilisation contre une réduction de l'activité lipolytique dans une concentration comprise entre 1 et 10 % en poids, de préférence entre 2 et 5 % en poids, sur la base du mélange d'enzymes digestives.

**17.** Préparations pharmaceutiques selon l'une des revendications 11 à 16, **caractérisées en ce que** la pancréatine qu'elles contiennent est une pancréatine prétraitée par autolyse.

**18.** Kit de préparation de solutions aqueuses de mélanges d'enzymes digestives, sans développement de germes, stabilisées contre une réduction de l'activité lipolytique, et appropriées pour l'introduction en continu au moyen de sondes, dans le tractus gastro-intestinal, **caractérisé en ce qu'**il contient comme composantes :

a) une préparation pharmaceutique solide, soluble dans l'eau et sans développement de germes, d'un mélange d'enzymes digestives contenant des lipases et des protéases, qui peut éventuellement contenir une quantité de lipides complexes selon l'une des revendications 1 à 9 suffisante pour la stabilisation contre une réduction de l'activité lipolytique de la solution aqueuse à préparer,

et

b) une quantité suffisante pour la préparation de la solution aqueuse d'un solvant aqueux sans développement de germe, qui peut contenir, en plus de l'eau, des sels et auxiliaires physiologiquement compatibles, et, dans le cas où la préparation pharmaceutique solide nommée en a) ne contient pas une quantité de lipides complexes suffisante pour stabiliser la solution aqueuse à préparer contre une réduction de l'activité lipolytique, une quantité supplémentaire de lipides complexes selon l'une des revendications 1 à 9 suffisante pour une stabilisation contre une réduction de l'activité lipolytique de la solution aqueuse à préparer.

**19.** Kit selon la revendication 18, **caractérisé en ce que** la préparation solide a) représente un mélange d'enzymes digestives lyophilisé, contenant des lipases et des protéases, et qui contient le cas échéant des lipides complexes.

**20.** Kit selon la revendication 19, **caractérisé en ce que** le mélange d'enzymes digestives lyophilisé contient de la pancréatine.

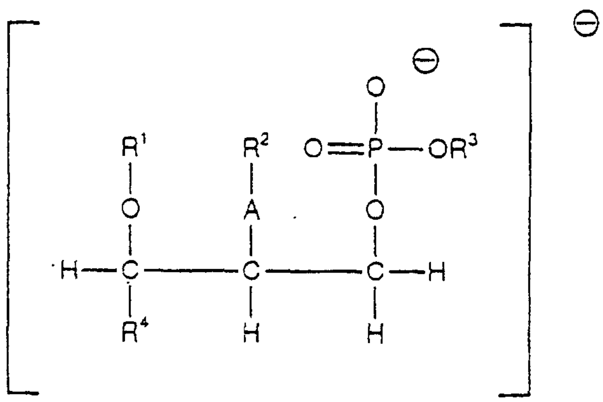

I

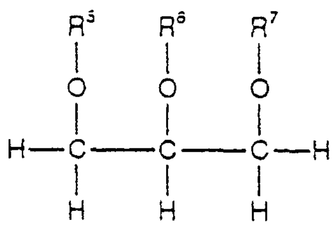

II